(19) Europäisches Patentamt
European Patent Office
Office européen des brevets

(11) Publication number: 0 266 966 A2

(12) **EUROPEAN PATENT APPLICATION**

(21) Application number: 87309543.4

(22) Date of filing: 29.10.87

(51) Int. Cl.⁴: C07C 179/18

(30) Priority: 06.11.86 JP 262759/86

(43) Date of publication of application: 11.05.88 Bulletin 88/19

(84) Designated Contracting States: DE FR GB IT NL

(71) Applicant: NIPPON OIL AND FATS COMPANY, LIMITED
10-1, Yuraku-cho 1-chome
Chiyoda-ku Tokyo(JP)

(72) Inventor: Takada, Jun
9 Aza-Nishitora Kanasawa
Chita City Aichi Pref.(JP)
Inventor: Banno, Shigeki
2-82 Aza-Kusunoki Taketoyo-Cho
Chita-Gun Aichi Pref.(JP)

(74) Representative: Sheader, Brian N. et al
ERIC POTTER & CLARKSON 27 South Street
Reading Berkshire, RG1 4QU(GB)

(54) **Novel peroxymonocarbonate.**

(57) A peroxymonocarbonate represented by the following general formula:

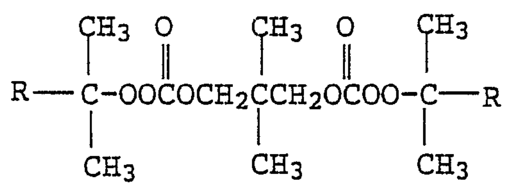

wherein R represents a member selected from the group consisting of an alkyl group having 1-5 carbon atoms, cyclohexyl group, cyclohexyl group having a substituent of an alkyl group having 1-4 carbon atoms, phenyl group and phenyl group having a substituent of an alkyl group having 1-3 carbon atoms, is excellent as crosslinking agents for rubber and thermoplastics, as a polymerization initiator for vinyl monomer, and as a hardening agent for unsaturated polyester resin.

EP 0 266 966 A2

## NOVEL PEROXYMONOCARBONATE

The present invention relates to a novel peroxymonocarbonate useful as an initiator for radical polymerization and a crosslinking agent for elastomer.

Peroxide is generally used as a polymerization initiator for unsaturated monomers, such as vinyl chloride, ethylene, styrene and the like, as crosslinking agents for elastomers, such as ethylene-propylene rubber, styrene-butadiene rubber, silicone rubber and the like, and for thermoplastics, such as polyethylene, poly(ethylene-vinyl acetate) and the like, and a hardening agent for unsaturated polyester resin.

For example, Japanese Patent Application Publication No. 46-15,086 discloses that a peroxymonocarbonate having an alkyl group having 8-16 carbon atoms is used as a crosslinking agent for polyethylene or poly(ethylene-vinyl acetate).

In general, it is known that peroxymonocarbonate is not influenced by fillers and enables to proceed a crosslinking reaction at a high rate. Further, it is known that peroxide, such as dicumyl peroxide, is not deteriorated in its crosslinking performance even in the presence of fillers, such as carbon black and the like, but the crosslinking reaction of a polymer in the presence of dicumyl peroxide is low in the crosslinking rate. Further, it is known that peroxides, such as tert.-butyl peroxybenzoate and benzoyl peroxide, which are crosslinking agents capable of enabling a crosslinking reaction of a polymer to proceed at a lower temperature, are apt to be influenced by a filler and the like.

Peroxymonocarbonates are useful crosslinking agents as described above, but they have the following drawbacks. That is, short chain peroxymonocarbonate is highly volatile, and hence the short chain peroxymonocarbonate is volatilized into air during its kneading together with elastomer or thermoplastics, or even after the short chain peroxymonocarbonate and the elastomer or thermoplastics have been formed into a compound through the kneading, and the crosslinking efficiency of the short chain peroxymonocarbonate is low. Moreover, there are dangerous problems in the use of the short chain peroxymonocarbonate, for example, the volatilized short chain peroxymonocarbonate ignites under the condition for carrying out the above described kneading to cause a fire.

Long chain peroxymonocarbonates, for example, peroxides disclosed in Japanese Patent Application Publication No. 46-15,086 and U.S. Patent No. 4,526,726 are free from the problems due to volatility, but they are small in the amount of active groups (peroxide groups) which occupy in the molecule, and are poor in the crosslinking efficiency per unit weight of the peroxide.

The object of the present invention is to provide a novel bifunctional peroxymonocarbonate which is free from the drawbacks of conventional peroxymonocarbonates.

That is, the feature of the present invention is the provision of a novel peroxymonocarbonate represented by the following general formula:

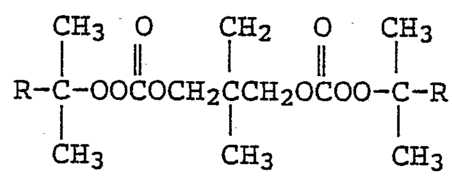

wherein R represents a member selected from the group consisting of an alkyl group having 1-5 carbon atoms, cyclohexyl group, cyclohexyl group having a substituent of an alkyl group having 1-4 carbon atoms, phenyl group and phenyl group having a substituent of an alkyl group having 1-3 carbon atoms.

The novel peroxymonocarbonates according to the present invention include 2,2-dimethyl-1,3-bis(t-butylperoxycarboxy)propane, 2,2-dimethyl-1,3-bis(t-hexylperoxycarboxy)propane, 2,2-dimethyl-1,3-bis-(1,1,3,3-tetramethylbutylperoxycarboxy)propane, 2,2-dimethyl-1,3-bis(cumylperoxycarboxy)propane, 2,2-dimethyl-1,3-bis(p-isopropylcumylperoxycarboxy)propane, 2,2-dimethyl-1,3-bis(m-isopropylcumylperoxycarboxy)propane, 2,2-dimethyl-1,3-bis(2-(4-methylcyclohexyl)isopropylperoxycarboxy)propane and the like.

The peroxymonocarbonate of the present invention can be produced under substantially the same reaction condition as that in the production of commonly known peroxymonocarbonate.

That is, the peroxymonocarbonate of the present invention can be obtained by reacting potassium salt of tertiary hydroperoxide with 2,2-dimethylpropane 1,3-dichloroformate (I) as illustrated in the following equation:

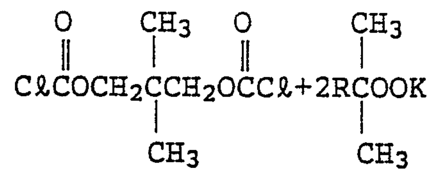

( I )

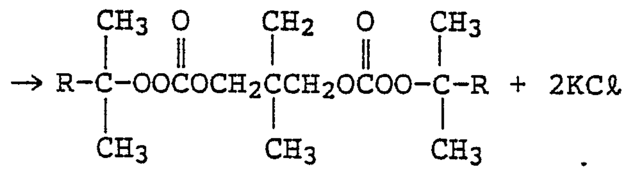

wherein R in the above described formulae has the same meaning as described above.

The tertiary hydroperoxides to be used in the present invention include tertiary-butyl hydroperoxide, tertiary-hexyl hydroperoxide, 1,1,3,3-tetramethylbutyl hydroperoxide, cumyl hydroperoxide, p-menthane hydroperoxide, p-isopropylcumyl hydroperoxide, m-isopropylcumyl hydroperoxide and the like.

In the above described reaction, the reaction temperature is -5°C~+35°C, preferably 10-20°C; the reaction time is 0.5-3 hours, preferably 1-2 hours; the molar ratio of the compound represented by the formula (I) to the tertiary hydroperoxide is within the range of 1/2 - 1/4, preferably 1/2.2 - 1/2.8; and the concentration of aqueous solution of potassium hydroxide is 5-30%, preferably 15-25%.

The peroxymonocarbonate of the present invention is a novel compound.

The peroxymonocarbonate of the present invention has a large molecular weight and has two functional groups per one molecule. Therefore, the peroxymonocarbonate of the present invention has such excellent properties that the activity per unit weight is high and the volatility is low.

Accordingly, in the crosslinking reaction of polymers, such as ethylene-propylene copolymer, polyethylene, poly(ethylene-vinyl acetate) and the like, the peroxymonocarbonate of the present invention has the following merits over conventional peroxymonocarbonates. That is, in the case of a comparison of the peroxymonocarbonate of the present invention with a conventional peroxymonocarbonate having substantially the same thermal decomposition temperature with each other, the peroxymonocarbonate of the present invention can exhibit the same crosslinking effect as the effect exhibited by the conventional peroxymonocarbonate even by the use of the peroxymonocarbonate of the present invention in an amount smaller than the amount of the conventional peroxymonocarbonate. Further, in the case of a comparison of the peroxymonocarbonate of the present invention with a conventional peroxymonocarbonate having substantially the same activity with each other, the peroxymonocarbonate of the present invention has a volatility remarkably lower than that of the conventional peroxymonocarbonate, and therefore the peroxymonocarbonate of the present invention does not volatilize during its kneading together with a polymer or even after the peroxymonocarbonate of the present invention and the polymer are formed into a compound through the kneading, and the peroxymonocarbonate of the present invention exhibits a crosslinking efficiency higher than the efficiency exhibited by the conventional peroxymonocarbonate. Moreover, the peroxymonocarbonate of the present invention does not cause the environmental pollution and is low in the flammability due to its non-volatilizing property, and hence the peroxymonocarbonate of the present invention has a high safeness and is very valuable for industry.

The poroxymonocarbonate of the present invention, in addition to the use as a crosslinking agent, can be used as an initiator for the polymerization of vinyl monomers, such as ethylene, vinyl chloride, vinyl acetate, acrylonitrile, vinylidene chloride, acrylic acid ester and the like; and further can be used as a hardening agent for unsaturated polyester resin.

The following examples are given for the purpose of illustration of this invention and are not intended as limitations thereof.

Example 1

Into a four-necked flask equipped with a stirrer and a thermometer was charged 11.6 g of a 20% aqueous solution of potassium hydroxide, and then charged 53.4 g of t-butyl hydroperoxide (purity: 81%) while keeping the reaction temperature to not higher than 15°C, and further charged dropwise a mixed solution consisting of 46.3 g of 2,2-dimethylpropane 1,3-dichloroformate (purity: 99.2%) and 46.3 g of n-hexane under stirring while keeping the reaction temperature to 15±2°C. After the stirring was continued for 90 minutes at this temperature, an organic layer was separated from the reaction mass, washed twice with a 5% aqueous solution of sodium hydroxide, further washed with water until the organic layer became neutral, and then dried with anhydrous magnesium sulfate to obtain an n-hexane solution. n-Hexane was distilled off from the n-hexane solution under vacuum to obtain a white solid substance, and the white solid substance was recrystallized from methanol to obtain 47.2 g of a white powder having an active oxygen content of 9.50%.

The resulting white powder had the following characteristic values and was identified to be 2,2-dimethyl-1,3-bis(t-butylperoxycarboxy)propane represented by the formula of

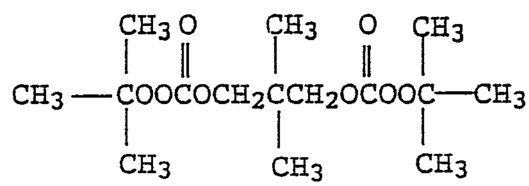

Purity measured by the iodometry: 99.9%
Infrared absorption spectrum:
865 $cm^{-1}$ (O-O)
1,795$^{-1}$ cm (C=O)
Nuclear magnetic resonance spectrum:
1.1 ppm (6H)
1.4 ppm (18H)
4.2 ppm (4H)

Example 2

The method described in Example 1 was repeated, except that 62.3 g of t-hexyl hydroperoxide (purity: 91%) was used in place of t-butyl hydroperoxide, to obtain 52.0 g of a liquid substance having an active oxygen content of 7.93%.

The resulting liquid substance had the following characteristic values, and was identified to be 2,2-dimethyl-1,3-bis(t-hexylperoxycarboxy)propane represented by the formula of

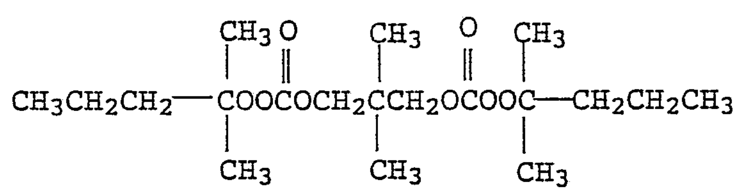

Purity measured by the iodometry: 97.3%
Infrared absorption spectrum:
865 $cm^{-1}$ (O-O)
1,795 $cm^{-1}$(C=O)
Nuclear magnetic resonance spectrum:
0.9 ppm (6H)
1.1 ppm (6H)
1.3 ppm (12H)

1.5 ppm (8H)
4.2 ppm (4H)

Example 3

The method described in Example 1 was repeated, except that 82.6 g of 1,1,3,3,-tetramethylbutyl hydroperoxide (purity: 85%) was used in place of t-butyl hydroperoxide, to obtain 58.3 g of a liquid substance having an active oxygen content of 6.78%.

The resulting liquid substance had the following characteristic values, and was identified to be 2,2-dimethyl-1,3-bis(1,1,3,3-tetramethylbutylperoxycarboxy)propane represented by the formula of

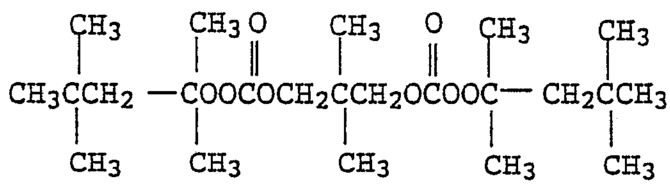

Purity measured by the iodometry: 95.1%
Infrared absorption spectrum:
863 $cm^{-1}$ (O-O)
1,795 $cm^{-1}$ (C=O)
Nuclear magnetic resonance spectrum:
1.0 ppm (18H)
1.1 ppm (6H)
1.4 ppm (12H)
1.6 ppm (4H)
4.2 ppm (4H)

Example 4

The method described in Example 1 was repeated, except that 91.3 g of cumene hydroperoxide (purity: 80%) was used in place of t-butyl hydroperoxide, to obtain 70.9 g of a white powder having an active oxygen content of 6.82%.

The resulting white powder had the following characteristic values, and was identified to be 2,2-dimethyl-1,3-bis(cumylperoxycarboxy)propane represented by the formula of

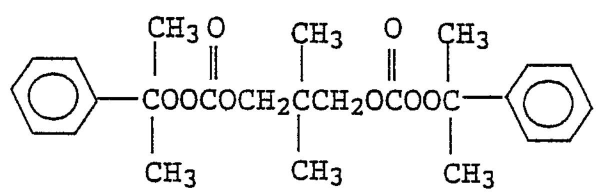

Purity measured by the iodometry: 98.2%
Infrared absorption spectrum:
867 $cm^{-1}$ (O-O)
1,805 $cm^{-1}$ (C=O)
Nuclear magnetic resonance spectrum:
1.1 ppm (6H)
1.6 ppm (12H)
4.2 ppm (4H)
7.6 ppm (10H)

Example 5

The method described in Example 1 was repeated, except that 166.5 g of m-diisopropylbenzene hydroperoxide (purity: 56%) was used in place of t-butyl hydroperoxide, to obtain 61.9% of a liquid substance having an active oxygen content of 5.48%.

The resulting liquid substance had the following characteristic values, and was identified to be 2,2-dimethyl-1,3-bis(m-isopropylcumylperoxycarboxy)propane represented by the formula of

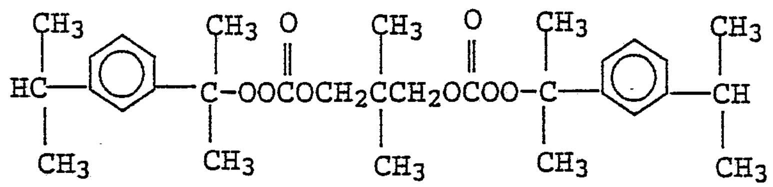

Purity measured by the iodometry: 93.2%
Infrared absorption spectrum:
865 $cm^{-1}$ (O-O)
1,805 $cm^{-1}$ (C=O)
Nuclear magnetic resonance spectrum:
1.1 ppm (6H)
1.3 ppm (12H)
1.6 ppm (12H)
2.9 ppm (2H)
4.2 ppm (4H)
7.2 ppm (8H)

Example 6

In 1,000 mℓ of styrene was dissolved 0.01 mol of 2,2-dimethyl-1,3-bis(t-butylperoxycarboxy)propane synthesized in Example 1, and 5 mℓ of the resulting solution was charged into an ampule of 10 mℓ capacity. Air remaining in the ampule was replaced with nitrogen gas, and then the ampule was sealed. The sealed ampule was immersed in an oil bath kept at 100°C for 6 hours to polymerize styrene, and the liquid reaction mass was poured into a large amount of methanol to precipitate polystyrene. The resulting polystyrene was separated through filtration and then dried under vacuum. The weight of the dried polystyrene was measured, and the conversion of styrene was calculated from the weight of the polystyrene according to the ordinary method.

Further, the molecular weight of the resulting polystyrene was measured by the gel permeation chromatography.

As the result, it was found that the conversion of styrene was 78.7% and the weight-average molecular weight of the resulting polystyrene was 382,000.

Comparative example 1

Styrene was polymerized in the same manner as described in Example 6, except that t-butylperoxy isopropylcarbonate was used in place of 2,2-dimethyl-1,3-bis(t-butylperoxycarboxy)propane used in Example 6, and the conversion of styrene and the molecular weight of the resulting polystyrene were measured in the same manner as described in Example 6.

As the result, it was found that the conversion of styrene was 68.7% and the weight-average molecular weight of the resulting polystyrene was 311,000.

It is clear from the comparison of Example 6 with Comparative example 1 that the peroxymonocarbonate of the present invention is higher than the conventional peroxymonocarbonate in the activity as a polymerization initiator.

Example 7

A mixture of 100 g of ethylene-propylene-diene rubber (EPDM), 0.01 mol of 2,2-dimethyl-1,3-bis(t-butylperoxycarboxy)propane synthesized in Example 1, 40 g of HAF carbon black, 1 g of stearic acid and 5 g of zinc oxide was kneaded and extruded into a sheet having a thickness of about 2 mm by means of two rolls.

The crosslinking property of the above used peroxymonocarbonate was measured in the following manner. That is, the resulting sheet was kept at room temperature in an open state for 2 hours and 10 days after the kneading. An oscillating disc rheometer was rotated at 160°C in each of the above treated sheets, and the maximum torque ($T_{max}$) for the rotation and the time ($T_{90\text{-}time}$) required for increasing the torque to 90% of the maximum torque were measured.

The obtained results are shown in Table 1.

Examples 8 and 9 and Comparative example 2

The experiment of Example 7 was repeated, except that 2,2-dimethyl-1,3-bis(1,1,3,3-tetramethylbutylperoxycarboxy)propane was used (Example 8), 2,2-dimethyl-1,3-bis(cumylperoxycarboxy)propane was used (Example 9), or a conventional peroxide of t-butylperoxy isopropylcarbonate was used (Comparative example 2) in place of 2,2-dimethyl-1,3-bis(t-butylperoxycarboxy)propane used in Example 7.

The obtained results are shown in the Table 1.

Table 1

| | After 2 hours | | After 10 days | |
|---|---|---|---|---|
| | $T_{max}$ (Kg-cm) | $T_{90\text{-}time}$ (min) | $T_{max}$ (Kg-cm) | $T_{90\text{-}time}$ (min) |
| Example 7 | 41.3 | 14.5 | 40.9 | 14.6 |
| Example 8 | 39.6 | 13.8 | 39.0 | 13.2 |
| Example 9 | 43.6 | 14.0 | 43.7 | 14.3 |
| Comparative example 2 | 34.1 | 14.0 | 26.9 | 16.4 |

It is clear from the results shown in Table 1 that the rubber treated with the peroxymonocarbonate of the present invention has a large $T_{max}$, and the peroxymonocarbonate of the present invention has a high activity due to the presence of two peroxy groups in one molecule. Further, comparison of the crosslinking property of the peroxymonocarbonate at 2 hours after the kneading with that at 10 days after the kneading shows as follows. In the rubber treated with the peroxymonocarbonate of the present invention, there are substantially no change in the values of $T_{max}$ and $T_{90\text{-}time}$, respectively. However, in the rubber treated with the conventional peroxymonocarbonate (Comparative example 2), the value of $T_{max}$ after 10 days is lower than that of $T_{max}$ after 2 hours, and the value of $T_{90\text{-}time}$after 10 days is longer than that of $T_{90\text{-}time}$ after 2 hours. This phenomenon is due to the evaporation of the conventional peroxymonocarbonate. Such change does not occur in the use of the peroxymonocarbonate of the present invention as described above due to its low volatility.

## Claims

A peroxymonocarbonate represented by the following general formula:

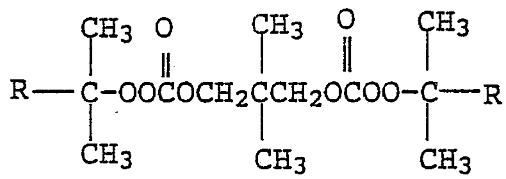

wherein R represents a member selected from the group consisting of an alkyl group having 1-5 carbon atoms, cyclohexyl group, cyclohexyl group having a substituent of an alkyl group having 1-4 carbon atoms, phenyl group and phenyl group having a substituent of an alkyl group having 1-3 carbon atoms.